# EUROPÄISCHE PATENTANMELDUNG

(11) **EP 2 824 173 A1**
(43) Veröffentlichungstag der Anmeldung: **14.01.2015**
(21) Anmeldenummer: 14176565.1
(22) Anmeldetag: 10.07.2014
(51) Int. Cl.: C12M 1/107, C12M 1/00, C12M 1/26, C12M 1/34, C12M 1/36

(54) **Fermenter zur Erzeugung von Biogas aus Biomasse**

(30) Priorität: 12.07.2013 DE 102013107432
(71) Anmelder: Lutz, Peter, 81925 Unterföhring (DE)
(72) Erfinder: Lutz, Peter, 81925 Unterföhring (DE)
(74) Vertreter: Alber, Norbert

(57) **Zusammenfassung**

Die im vorderen Bereich eines Fermenters **(1)** bei der Trockenfermentation quer verlaufende Sammelrinne **(10)** für Perkolat wird erfindungsgemäß direkt unterhalb der Stützvorrichtung **(6)** angeordnet, die den Druck der Biomasse **(7)** aufnimmt. Dadurch wird vor allem ein Verstopfen des abdeckenden Gitters **(14)** der Sammelrinne **(10)** durch Biomasse **(7)** vermieden, und dennoch ein ausreichender Abfluss an Perkolat in die Sammelrinne **(10)** sichergestellt.

## Beschreibung

### I. Anwendungsgebiet

Die Erfindung betrifft einen Fermenter zur Erzeugung von Biogas aus Biomasse.

### II. Technischer Hintergrund

Heute wird Biomasse, beispielsweise Mais in frischer Form oder als Silage, auf zwei grundsätzlich unterschiedliche Arten anaerob vergoren:
- Einerseits im Nassgär-Verfahren, bei dem die Biomasse in Form einer pumpfähigen Schlempe vorliegt,
- Andererseits im hier vorliegenden Feststoff-Verfahren, bei der die Biomasse, nur durch das Animpfen mit bereits vergorenem Material, in die Fermenter eingebracht wird.

Bei diesem Feststoff-Verfahren, dem so genannten Trockenfermentationsverfahren, sammelt sich Sickerflüssigkeit, das so genannte Perkolat, am Boden des Fermenters, welches aus der in der Biomasse enthaltenen Flüssigkeit besteht, die durch das Eigengewicht der Biomasse heraus gepresst wird, und die mit Fortschritt des Umsetzungsprozesses zusätzlich biologisch aktive Gär-Stoffe enthält.

Dabei ist es üblich, dieses Perkolat aus dem Bodenbereich der Fermenter abzupumpen und wieder auf der Oberseite der Biomasse im Fermenter zu versprühen.

Dadurch wird das Perkolat gleichmäßig über das gesamte Volumen der Biomasse im Fermenter verteilt. Gleichzeitig kann eine Temperaturregelung im Fermenter mittels des zirkulierenden Perkolates erreicht werden, indem dieses vor der Rückführung nach Bedarf beheizt wird.

Dabei unterscheiden sich sowohl die Menge als auch eventuell die Inhaltsstoffe des einem Fermenter zuzuführenden Perkolates abhängig davon, in welchem Zustand der Umsetzungsprozesse sich befindet, welche Art von ursprünglicher Biomasse er enthält und aufgrund von weiteren Faktoren.

Bisher wurde das Perkolat am dichten, nicht flüssigkeitsdurchlässigen, befahrbaren Boden des Fermenters, welcher ein Gefälle zur Ladeöffnung hin aufwies und höchstens dorthin führende Längsrillen im Boden, mithilfe einer quer nahe der Ladeöffnung verlaufenden Sammelrinne gesammelt. Damit die Sammelrinne nicht verstopft und das Perkolat möglichst ungehindert in die Sammelrinne hinein fließen kann, ist es bereits bekannt, die Sammelrinne zwischen der dicht verschließenden Klappe und der meist vorhandenen, weiter innen liegenden Stützvorrichtung für die angehäufte Biomasse anzuordnen.

Es kommt auch vor, dass sich das Perkolat im Fermenter aufstaut mit der Folge, dass die Biomasse auf dem aufgestauten Perkolat schwimmt und über die Oberkante der Stützvorrichtung, die nicht bis zur Decke des Fermenters reicht, nach vorne in den Zwischenraum zwischen Stützvorrichtung und verschließender z.B. Klappe herab fällt, und damit auf die Sammelrinne für das Perkolat, die dadurch verstopft wird.

### III. Darstellung der Erfindung

### a) Technische Aufgabe

Es ist daher die Aufgabe gemäß der Erfindung, einen Fermenter und insbesondere eine Perkolat-Sammeleinrichtung im Fermenter zur Verfügung zu stellen, die auch im Fall eines aufgestauten Perkolates nicht verstopft

### b) Lösung der Aufgabe

Diese Aufgabe wird durch die Merkmale des Anspruchs 1 gelöst. Vorteilhafte Ausführungsformen ergeben sich aus den Unteransprüchen.

Durch die Anordnung der Perkolat-Sammeleinrichtung in der Aufsicht betrachtet unter der Stützvorrichtung wird die Sammeleinrichtung zumindest teilweise durch die darüber angeordnete Stützvorrichtung abgedeckt, sodass sich in diesem Bereich keine Biomasse auf der Sammeleinrichtung, die ja meistens eine mittels eines Gitters abgedeckte Sammelrinne ist, ablagern kann und die Sammeleinrichtung verstopfen kann.

Dies gilt erst recht, wenn sich die Sammeleinrichtung ausschließlich unterhalb der Stützvorrichtung befindet.

Die Sammeleinrichtung kann sich auch - betrachtet in horizontaler Verlaufsrichtung der Stützvorrichtung - nach hinten, also in Richtung Biomasse, über die Hinterkante der Stützvorrichtung hinaus erstrecken. Es lagert sich dort natürlich die Biomasse auf diesen Teil der Sammeleinrichtung ab, sodass in diesem Bereich das Einsickern des Perkolats erschwert wird, aber dennoch nicht unmöglich wird. Zusätzlich steht aber zum leichten Einsickern des Perkolats in die Sammeleinrichtung der Bereich unmittelbar unterhalb der Stützvorrichtung zur Verfügung.

Die Sammeleinrichtung ist vorzugsweise eine quer, insbesondere im rechten Winkel, zur Einfahrrichtung in den offenen Fermenter, verlaufende Rinne im flüssigkeitsdichten Boden, die von einem Gitter, das entfernbar und insbesondere nur aufgelegt ist, abgedeckt ist und insbesondere einen Abfluss für das Perkolat nach außerhalb des Fermenters aufweist.

Die Stützvorrichtung, die im eingebauten Zustand etwa vertikal im Fermenter steht und auch meist in vertikaler Richtung eingehängt wird, ist wenigstens teilweise für Perkolat durchlässig. Sie kann auf der Oberseite der PerkolatSammeleinrichtung aufsitzen oder in einem geringen Abstand von **2** mm bis **20** mm, insbesondere von **5** mm bis **10** mm darüber enden. Ein größerer Abstand ist nachteilig, da sich dann Biomasse durch den Abstand hindurch in den Bereich unterhalb der Stützvorrichtung gelangen und wiederum die Perkolat-Sammeleinrichtung verstopfen kann.

Die Stützvorrichtung besteht in der Regel aus einem stabilen Stützrahmen aus Metall, der zumindest einseitig auf der Rückseite, also zur Biomasse hin, beplankt ist, meist mit Holz-Planken, die vertikal verlaufend und im Abstand zueinander auf dem Stützrahmen montiert sind. Das Perkolat hat also verschiedene Möglichkeiten, in die Perkolat-Sammeleinrichtung zu gelangen:

Das meiste Perkolat wird durch den Abstand zwischen den vertikalen Planken über die gesamte Höhe der Stützvorrichtung durch die Beplankung in die Stützvorrichtung hineinsickern und beispielsweise an den Planken nach unten laufen in die Sammeleinrichtung. Zu diesem Zweck sollte auch der Abstand zwischen den Planken sich im Bereich von **2** mm bis **20** mm, vorzugsweise zwischen **5** mm und **10** mm, bewegen, um das Eindringen von Biomasse durch die Beplankung in die Stützvorrichtung hinein zu verhindern.

Ein weiterer Weg für das Perkolat besteht in dem meist vorhandenen vertikalen Abstand zwischen der Unterkante der Beplankung der Stützvorrichtung und der Oberseite der Perkolat-Sammeleinrichtung, deren Oberkante ja in der Regel mit der Oberkante des Bodens des Fermenters fluchtet, um Beschädigungen beim Überfahren mit schwerem Gerät, z. B. einem Radlader, der mehr als 3 t wiegen kann, zu vermeiden.

Die Beplankung ragt dabei über das untere Ende des Stützrahmens nach unten vor, sodass ein freies Abtropfen des Perkolats von den unteren Enden der Planken der Stützvorrichtung erfolgen kann.

Vorzugsweise ist auch die der Schließvorrichtung zugewandte Vorderseite des Stützrahmens der Stützvorrichtung ebenfalls beplankt mit Abstand zwischen den Planken, um Biomasse, welche durch die Abstände der rückseitigen Beplankung hindurch eventuell eingedrungen ist, zurückzuhalten.

Die vorderseitige Beplankung dient zusätzlich der Ableitung von Perkolat, welches durch die rückseitige Beplankung hindurch entlang des Stützrahmens weitergesickert ist und leitet das Perkolat an der Vorderseite der Stützvorrichtung nach unten in die Perkolat-Sammeleinrichtung.

Zusätzlich sind in allen quer verlaufenden Streben des Stützrahmens vertikale Durchgangslöcher vorhanden, um das darauf stehende Perkolat nach unten ableiten zu können.

Sofern die Sammeleinrichtung nicht nach hinten über das hintere Ende der Stützvorrichtung in Richtung Biomasse übersteht, ist die Stützvorrichtung mindestens so dick wie die Breite der Perkolat-Sammeleinrichtung. Die Beplankung der Stützvorrichtung kann auch nach vorne und/oder hinten über die Breite der Perkolat-Sammeleinrichtung vorstehen.

Dadurch wird die Breite der Sammelrinne so gering wie möglich gehalten, was die Beschädigungsgefahr beim Überfahren beim Be- und Entladen des Fermenters reduziert.

Die Schließvorrichtung und ebenso die Stützvorrichtung müssen sich nicht in Querrichtung über die gesamte Frontfläche des Fermenters erstrecken, sondern können auch nur eine demgegenüber verringerte Ladeöffnung verschließen.

Die Schließvorrichtung ist in der Regel eine an der Oberkante der Ladeöffnung schwenkbar befestigte und nach oben öffnende Klappe, da diese im geöffneten Zustand die Be- und Entladetätigkeit am wenigsten beeinträchtigt und in geöffnetem Zustand kaum freien Raum neben dem Fermenter benötigt.

Die Sammeleinrichtung, insbesondere die in Querrichtung verlaufende, unter der Stützvorrichtung im Boden angeordnete Sammelrinne, kann zusätzlich mit in Längsrichtung im Boden des Fermenters ausgebildeten LängsSammelrinnen für das Perkolat in Verbindung stehen, die sich im Wesentlichen über die gesamte Länge des Fermenters erstrecken. In den LängsSammelrinnen liegt insbesondere einerseits ein Heizrohr, welches von einem Heizmedium durchströmt ist, um vor allem das Perkolat aufzuheizen, und die Längs-Sammelrinnen können insbesondere jeweils von einem Gitter abgedeckt sein.

### c) Ausführungsbeispiele

Ausführungsformen gemäß der Erfindung sind im Folgenden beispielhaft näher beschrieben. Es zeigen:
- Fig. **1:**: eine perspektivische Ansicht eines geöffneten Fermenters,
- Fig. **2:**: eine seitliche Schnittdarstellung des Frontteiles eines geöffneten Fermenters,
- Fig. **3**a-c:: vergrößerte seitliche Schnittdarstellungen im Frontbereich eines Fermenters.

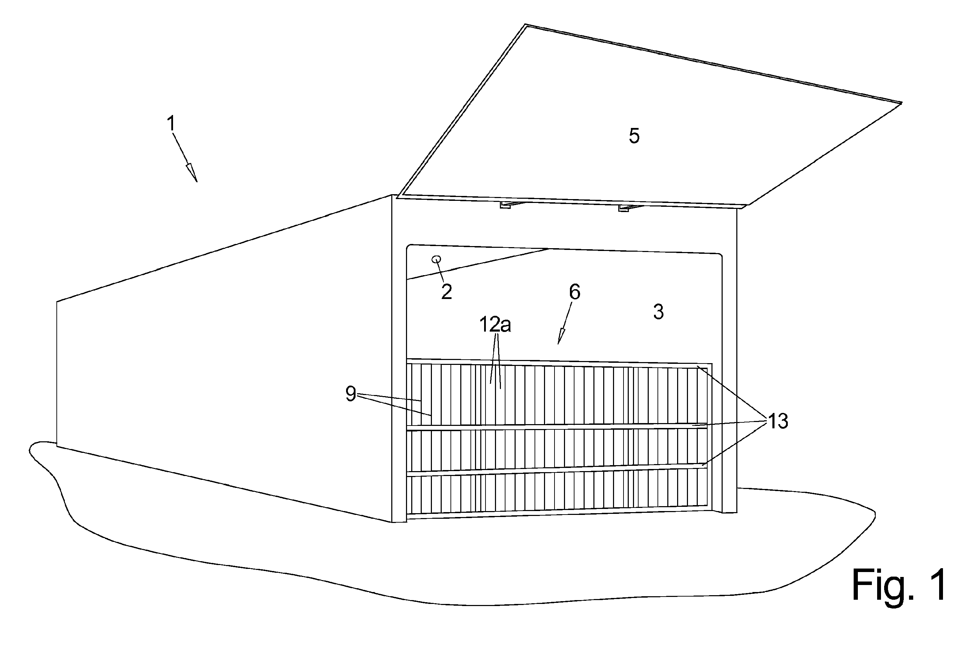

**Figur 1** zeigt einen Fermenter **1** in Form eines länglichen liegenden Quaders, dessen Haupterstreckungsrichtung die Längsrichtung ist, der in der Regel aus Beton gegossen ist und in der vorderen Stirnfläche eine großflächige Ladeöffnung **3** aufweist, die mittels der hier im geöffneten Zustand dargestellten Schließvorrichtung **5** in Form einer Klappe gasdicht und flüssigkeitsdicht verschlossen werden kann. Das in dem Fermenter **1** aus der dort eingelagerten Biomasse **7** entstehende Biogas wird über die Biogas-Entnahmeöffnung **2** abgeführt.

Im Abstand in Einfahrrichtung, der Längsrichtung, geringfügig hinter der Frontfläche, also der Ladeöffnung **3,** des Fermenters **1** ist in Figur **1** die Stützvorrichtung **6** für die dahinter aufgeschüttete Biomasse **7** montiert, die sich über die gesamte Breite der Ladeöffnung **3** erstrecken kann, also von der linken zur rechten Seitenwand des Fermenters **1** und sich vom Boden aus über etwa die Hälfte der Höhe des Fermenters **1** erstreckt.

Für das Beladen des Fermenters **1** wird diese Stützvorrichtung **6** natürlich entfernt, sodass zum Beispiel mittels eines z. B. über **3** t schweren Radladers Biomasse **7** ins Innere des Fermenters **1** gefahren und dort abgeladen werden kann, beginnend vom hinteren Ende nach vorne und so hoch aufgeschüttet wie möglich. Erst kurz vor Erreichen des vorderen Endes des Fermenters **1** mit der Füllung wird die Stützvorrichtung **6** eingebaut und zum Schluss noch Biomasse **7** über die Oberkante der Stützvorrichtung **6** in das Innere des Fermenters **1** abgekippt.

Ein Teil des Gewichts der Biomasse drückt somit gegen die nach innen weisende Rückseite der Stützvorrichtung **6,** sodass dieser Druck nicht von der Schließvorrichtung **5** aufgenommen werden muss, die dadurch sehr viel leichter gasdicht und flüssigkeitsdicht gehalten werden kann.

In Figur **1** ist die Stützvorrichtung **6** dargestellt bestehend aus einem Stützrahmen **13,** der lediglich auf der innen liegenden Rückseite mit einer Beplankung **12**a versehen ist, die aus vertikal verlaufenden Planken **12**a, b besteht, die mit seitlichem Abstand **9** zueinander auf dem Stützrahmen **13** montiert sind.

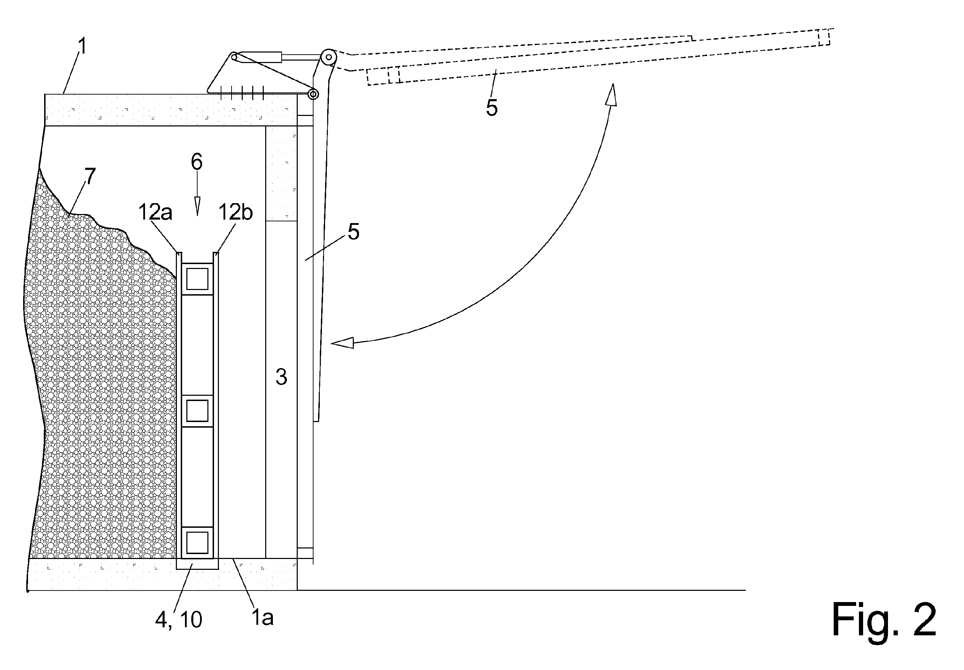

**Figur 2** zeigt den Frontbereich des Fermenters **1** in der Seitenansicht geschnitten und bei geschlossener Schließvorrichtung **5** und im befüllten Zustand, also auch mit eingebauter Stützvorrichtung **6.**

In diesem Fall ist die Stützvorrichtung **6** auf beiden Seiten des Stützrahmens **13** mit einer Beplankung **12**a, b versehen, wiederum jeweils mit im Abstand **9** zueinander angeordneten, vertikal verlaufenden Planken **12**a,b.

Unterhalb der Stützvorrichtung **6** befindet sich eine Sammelrinne **10** für Perkolat eingelassen im flüssigkeitsdichten Boden **1**a des Fermenters **1,** die sich quer über vorzugsweise die gesamte Breite des Fermenters **1** erstreckt.

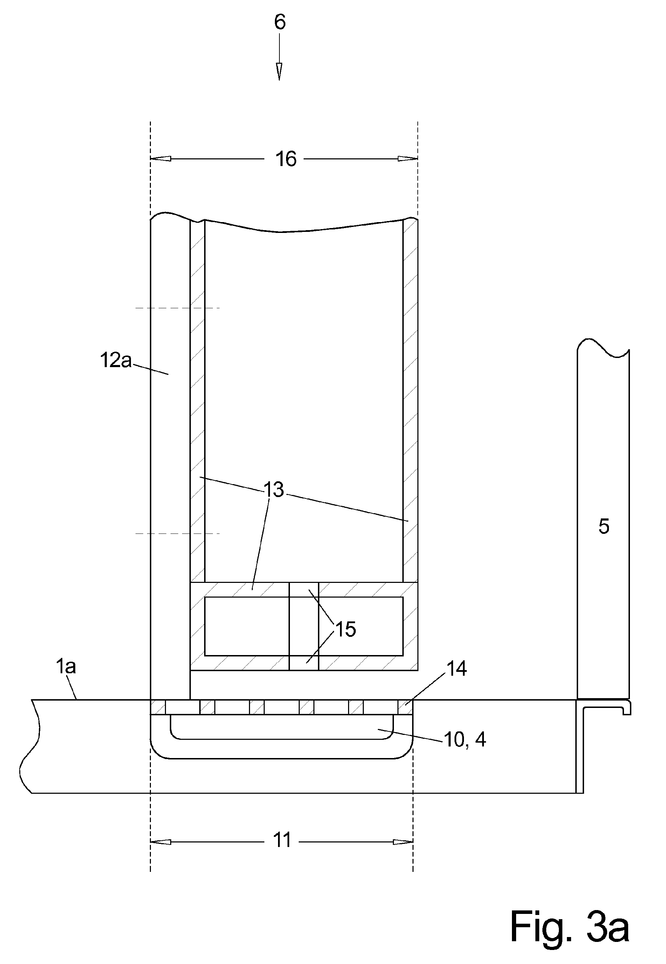

In den Figuren **3a** bis **3c** sind unterschiedliche Positionierungen und Breiten **11** der Sammelrinne **10** zur Stützvorrichtung **6** dargestellt:

In **Figur 3a** besteht die Stützvorrichtung **6** aus dem Stützrahmen **13** mit einer Beplankung **12**a lediglich auf der von der Schließvorrichtung **5** abgewandten Rückseite der Stützvorrichtung **6.**

Die Sammelrinne **10,** die üblicherweise mit einem insbesondere entfernbaren, z. B. nur auf die Seitenwände der Rinne aufgelegten Gitter **14** abgedeckt ist, befindet sich in der Aufsicht betrachtet mit Ihrer Breite **11** nur und ausschließlich unterhalb der Stützvorrichtung **6,** die somit eine Dicke **16** besitzt, die gleich groß oder größer ist wie die Breite **11** der Sammelrinne **10:**

Die Rückseite der Beplankung **12**a befindet sich oberhalb des rückseitigen Endes der Sammelrinne **10,** und die Frontseite des Stützrahmens **13** befindet sich oberhalb oder noch etwas vor dem vorderen Ende der Sammelrinne **10.**

Die Beplankung **12**a steht nach unten über das untere Ende des Stützrahmens **13** vor und sitzt auf dem Gitter **14** der Sammelrinne **10** auf, sodass an der Beplankung **12**a in den Abständen **9** zwischen den Planken nach unten laufendes Perkolat direkt in die Sammelrinne **10** hineinläuft.

Perkolat, welches durch die Beplankung **12**a hindurch dringt und entlang des Stützrahmens **13** nach unten läuft, kann durch vertikale Durchgangsöffnungen **15** in den querverlaufenden Streben des Stützrahmens **13** nach unten in die Sammelrinne **10** abfließen oder über die offene Vorderseite des Stützrahmens **13.**

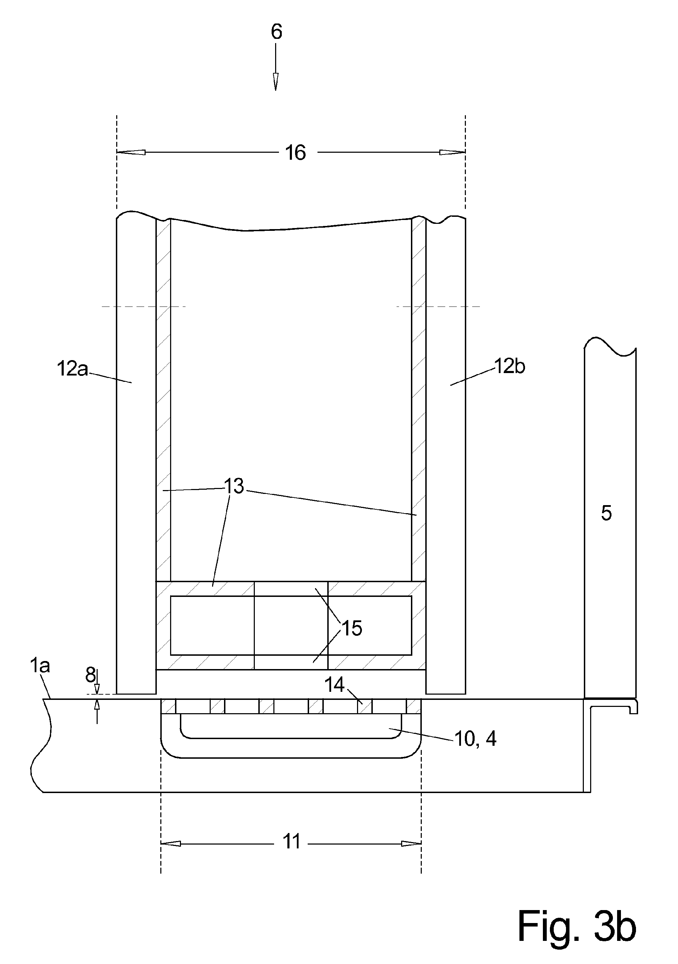

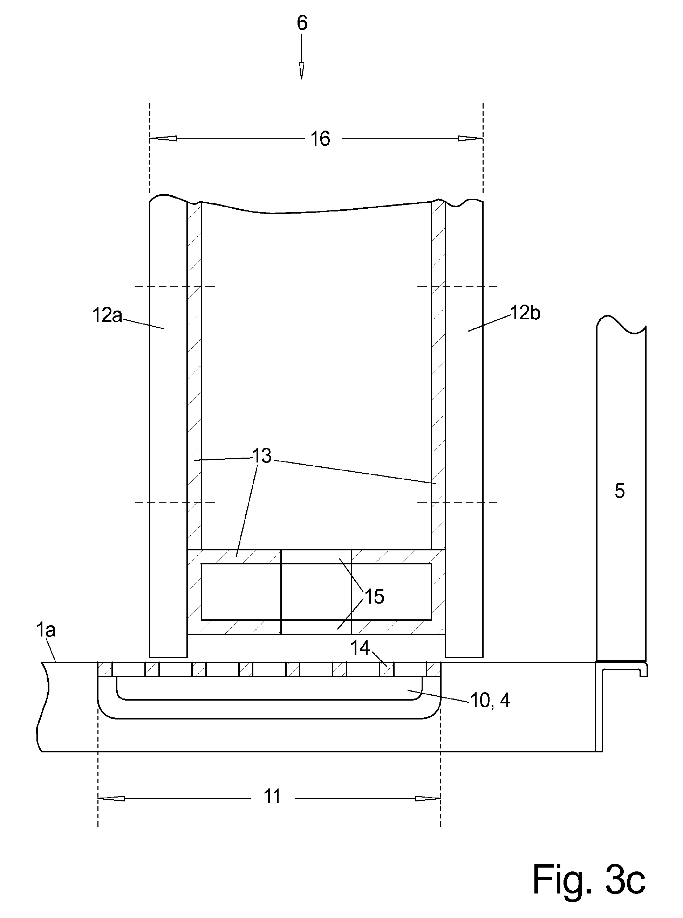

**Figur 3b** zeigt eine Anordnung, bei der im Unterschied zur Figur **3**a zum einen der Stützrahmen **13** beidseitig, also sowohl auf der Vorderseite als auch auf der Rückseite, eine Beplankung **12**a, b aus im vertikalem Abstand **8** nebeneinander auf dem Stützrahmen **13** befestigten Planken aufweist.

Die Dicke **16** der Stützvorrichtung **6** einschließlich der Beplankung **12**a, b ist in Figur 3b größer als die Breite **11** der Sammelrinne **10.**

Insbesondere sind die Beplankungen **12**a, b außerhalb der Breite der Sammelrinne **10** angeordnet. Zusätzlich enden aber die Beplankungen **12**a, b, insbesondere die rückseitige Beplankung **12**a, im Abstand **8** oberhalb des Bodens **1**a des Fermenters **1,** in den die Sammelrinne **13** bündig eingelassen ist. Dadurch kann durch diesen vertikalen Abstand **8** hindurch das aus der Biomasse **7** austretende Perkolat durch diesen Abstand **8** hindurch zur Sammelrinne **10** fließen, denn in aller Regel besitzt der Boden **1**a des Fermenters ein Gefälle vom hinteren Ende zur Sammeleinrichtung **4,** der Sammelrinne **10,** hin.

Sollte Biomasse über die Oberkante der Stützvorrichtung **6** hinweg nach vorne in den Zwischenraum zwischen Stützvorrichtung **6** und Schließvorrichtung **5** gefallen sein, kann das darin enthaltene Perkolat von dort unter dem Abstand der vorderen Beplankung **12**b hindurch zur Sammelrinne **13** abfließen. Die Anordnung der **Figur 3c** unterscheidet sich von derjenigen der Figur **3**b dadurch, dass die Sammelrinne **10** sich mit ihrer Breite **11** über die Rückseite der Stützvorrichtung **6** hinaus etwas nach hinten erstreckt, sodass an der rückseitigen Beplankung **12**a herablaufendes Perkolat direkt in die Sammelrinne **13** abfließen kann.

Die Breite **11** der Sammelrinne **10** kann in diesem Fall größer sein als die Dicke **16** der Stützvorrichtung **6.**

### BEZUGSZEICHENLISTE

- 1: Fermenter
- **1a**: Boden
- **2**: Biogas-Entnahmeöfifnung
- **3**: Ladeöffnung
- **4**: Perkolat-Sammeleinrichtung
- **5**: Schließvorrichtung
- **6**: Stützvorrichtung
- **7**: Biomasse
- **8**: (vert.) Abstand
- **9**: (horiz.) Abstand
- **10**: Sammelrinne
- **11**: Breite
- **12**a, b: Beplankung
- **13**: Stützrahmen
- **14**: Gitter
- **15**: vertikale Durchgangsöffnung
- **16**: Dicke

## Patentansprüche

1. Fermenter **(1)** zur Erzeugung von Biogas aus Biomasse **(7)** mit
- wenigstens einer im Wesentlichen vertikal ausgerichteten Ladeöffnung **(3)** zum Befüllen und Entnehmen der Biomasse **(7),**
- einer die Ladeöffnung **(3)** in geschlossenem Zustand gas- und flüssigkeitsdicht verschließenden Schließvorrichtung **(5),**
- einer im Abstand hinter der Schließvorrichtung **(5)** im Wesentlichen vertikal angeordneten Stützvorrichtung **(6)** für die dahinter liegende Biomasse **(7),**
- einer Perkolat-Sammeleinrichtung **(4)** im Boden **(1a)** des Fermenters **(1),**
**dadurch gekennzeichnet, dass**
die Perkolat-Sammeleinrichtung **(4)** in der Aufsicht betrachtet unter der Stützvorrichtung **(6),** insbesondere nur unter der Stützvorrichtung **(6),** angeordnet ist.

2. Fermenter **(1)** nach Anspruch **1,**
**dadurch gekennzeichnet, dass**
die Unterseite der Stützvorrichtung **(6)** im eingebauten Zustand in einem Abstand **(8)** oberhalb der Perkolat-Sammeleinrichtung **(4)** angeordnet ist, insbesondere in einem Abstand von **20** mm bis **2** mm, besser in einem Abstand von **10** mm bis **5** mm.

3. Fermenter (1) nach einem der vorhergehenden Ansprüche, **dadurch gekennzeichnet, dass**
die Perkolat-Sammeleinrichtung **(4)** eine nach oben offene, insbesondere von einem vorzugsweise abnehmbaren Gitter **(14)** abgedeckte, Sammelrinne **(10)** ist.

4. Fermenter **(1)** nach einem der vorhergehenden Ansprüche,
**dadurch gekennzeichnet, dass**
die Stützvorrichtung **(6)** wenigstens teilweise für Perkolat durchlässig ist, insbesondere eine vertikale Beplankung **(12a,** b) mit Abständen **(9)** zwischen den Planken zumindest auf der Seite der Biomasse **(7),** insbesondere auf beiden Seiten, aufweist.

5. Fermenter **(1)** nach einem der vorhergehenden Ansprüche,
**dadurch gekennzeichnet, dass**
die Stützvorrichtung **(6)** mindestens so dick ist wie die Breite **(11)** der Sammelrinne **(10),** insbesondere bei eingebauter Stützvorrichtung **(6)** die Beplankung **(12a,** b) außerhalb der Breite **(11)** der Sammelrinne **(10)** angeordnet ist.

6. Fermenter **(1)** nach einem der vorhergehenden Ansprüche,
**dadurch gekennzeichnet, dass**
die Beplankung **(12a,** b) auf einem stabilen Stützrahmen **(13)** aufgebracht ist, der insbesondere aus Metall besteht, und der in querverlaufenden Streben Durchgangsöffnungen **(15)** für das Perkolat aufweist.

7. Fermenter **(1)** nach einem der vorhergehenden Ansprüche,
**dadurch gekennzeichnet, dass**
die Stützvorrichtung **(6)** sich wenigstens über einen Teil der gesamten Breite des Fermenters **(1)** erstreckt, welche einer durch die Schließvorrichtung **(5)** verschließbaren Ladeöffnung **(3)** entspricht, insbesondere sich die Stützvorrichtung **(6)** über die gesamte Breite **(11)** des Fermenters **(1)** erstreckt.

8. Fermenter **(1)** nach einem der vorhergehenden Ansprüche,
**dadurch gekennzeichnet, dass**
die Schließvorrichtung **(5)** eine an der Oberkante der Ladeöffnung **(3)** schwenkbar befestigte Klappe ist.

9. Fermenter **(1)** nach einem der vorhergehenden Ansprüche,
**dadurch gekennzeichnet, dass**
die Oberseite der Sammelrinne **(10)** oder, falls ein abdeckendes Gitter **(14)** vorhanden ist, des Gitters **(14)** mit der Oberseite des Bodens **(1a)** des Fermenters **(1)** fluchtet.

10. Fermenter **(1)** nach einem der vorhergehenden Ansprüche,
**dadurch gekennzeichnet, dass**
die Sammelrinne **(10)** in Längsrichtung nach hinten über die Stützvorrichtung **(6)** maximal um die doppelte Dicke der Stützvorrichtung **(6),** besser nur um die Dicke der Stützvorrichtung **(6),** besser nur um die Hälfte der Dicke der Stützvorrichtung **(6),** vorsteht.
